# EUROPÄISCHE PATENTANMELDUNG

(11) **EP 0 761 814 A1**
(43) Veröffentlichungstag der Anmeldung: **12.03.1997**
(21) Anmeldenummer: 96112689.3
(22) Anmeldetag: 07.08.1996
(51) Int. Cl.: C12N 15/06, C07K 16/28, C07K 16/30, C12N 5/20, G01N 33/574, G01N 33/577, A61K 39/395

(54) **Antikörper 103 B2 gegen Peanut-Agglutinin(PNA)-bindendes Glykoprotein an Zelloberflächen**

(30) Priorität: 17.08.1995 DE 19530272
(71) Anmelder: Eberhard-Karls-Universität Tübingen Universitätsklinikum, 72076 Tübingen (DE)
(72) Erfinder: Bühring, Hans-Jörg, Dr., FACS-Labor, 72076 Tübingen (DE); Zannettino, Andrew, Highbury, SA 5089 (AU); Simmons, Paul J., Adelaide, SA 5061 (AU)
(74) Vertreter: Otten, Hajo, Dr.-Ing.

(57) **Zusammenfassung**

Die vorliegende Erfindung betrifft einen monoklonalen Antikörper, der spezifisch an das Peanut Agglutinin (PNA)-bindende Zelloberflächenglykoprotein MGC-24 bindet. Die Erfindung betrifft ferner Hybridomzellen, die einen derartigen Antikörper erzeugen, sowie ein Verfahren zur Herstellung solcher Hybridomzellen.

## Beschreibung

Die Erfindung betrifft einen Antikörper gegen Peanut Agglutinin (PNA)-bindendes Glykoprotein an Zelloberflächen.

Maligne Zellen von vielen menschlichen Tumoren besitzen an ihrer Zelloberfläche Peanut Agglutinin (PNA)-bindende Glykoproteine. Diese PNA-bindenden Glykoproteine bieten u.a. die Möglichkeit, Nachweisreagenzien und/oder therapeutisch wirksame Reagenzien direkt an die betreffenden Zellen heranzuführen und daran zu binden. Seit einigen Jahren bekannt sind die nach der inzwischen eingeführten MUC-Nomenklatur benannten Glykoproteine MUC1, MUC2 und MUC3. Ein kürzlich identifiziertes Zelloberflächen-Glykoprotein ist das Protein MGC-24, dessen Aminosäuresequenz und dazugehörige Nukleotidsequenz von Masuzawa, et al., J. BIOCHEM. 112, 609 - 615, (1992) vollständig aufgeklärt wurde. Dieses Glykoprotein MGC-24 eignet sich daher besonders gut als Angriffspunkt für eine gezielte zelluläre Diagnose bzw. Therapie.

Als Vermittler für ein dementsprechendes, zellulär zielgerichtetes Diagnostikum bzw. Therapeutikum kommt insbesondere ein Antikörper in Betracht, der spezifisch an dieses Glykoprotein MGC-24 bindet.

Ein solcher Antikörper kann sowohl mit einfachen Nachweisreagenzien wie Fluoreszenzfarbstoffen oder radioaktiven Stoffen als auch mit speziellen therapeutisch wirksamen Reagenzien gekoppelt sein.

Bisher ist jedoch nur ein polyklonaler und damit nur begrenzt verfügbarer und nicht identisch reproduzierbarer Antikörper bekannt, der zudem gegen eine modifizierte, nämlich deglykosilierte Form des MGC-24-Proteins gerichtet ist.

Mit einem derartigen Antikörper ist eine gezielte Behandlung bzw. ein gezielter Nachweis von MGC-24 tragenden Zellen u.a. im lebenden Organismus nicht möglich.

Der vorliegenden Erfindung liegt daher die Aufgabe zugrunde, einen Antikörper bereitzustellen, der spezifisch an das native, unmodifizierte Peanut Agglutinin (PNA)-bindende Zelloberflächenglykoprotein MGC-24 bindet und der in praktisch unbegrenzter Menge zur Verfügung steht.

Diese Aufgabe wird durch die Bereitstellung eines monoklonalen Antikörpers gelöst, der spezifisch an das native, unmodifizierte Peanut Agglutinin (PNA)-bindende Zelloberflächenglykoprotein MGC-24 bindet. Ein solcher monoklonaler Antikörper wird von Hybridomzellen produziert und freigesetzt, die nach dem Budapester Vertrag unter der Nummer DSM ACC 2221 bei der Deutschen Sammlung von Mikroorganismen und Zellkulturen GmbH, DSM, hinterlegt sind. Er ist mit der Bezeichnung 103B2 benannt.

Ein weiterer monoklonaler Antikörper, der spezifisch an das native, unmodifizierte Peanut Agglutinin (PNA)-bindende Zelloberflächenglykoprotein MGC-24 bindet, allerdings an ein anderes Epitop, wird von Hybridomzellen produziert und freigesetzt, die unter der Nummer DSM ACC 2222 bei der Deutschen Sammlung von Mikroorganismen und Zellkulturen GmbH, DSM, hinterlegt sind. Er ist mit der Bezeichnung 105A5 benannt. Dieser Antikörper ist Gegenstand der zeitgleich eingereichten deutschen Patentanmeldung DE 195 30 273.7 "Antikörper 105A5".

Mit dem erfindungsgemäßen Antikörper und dem Antikörper aus der parallelen Patentanmeldung wurde erstmals jeweils ein monoklonaler Antikörper bereitgestellt, der standardisiert reproduzierbar ist und somit potentiell unbegrenzt hergestellt werden kann, und der spezifisch an jeweils ein spezielles Epitop auf dem Zelloberflächenglykoprotein MGC-24 bindet.

Der erfindungsgemäße Antikörper ermöglicht eine gezielte Erkennung und Beeinflussung von Zellen, die eine extrazelluläre Domäne dieses Protein MGC-24 aufweisen. Er stellt damit ein bisher einzigartiges und vielseitig einsetzbares Mittel für den Arzt und Forscher dar, um einerseits solche Zellen nachzuweisen, und zwar sowohl in der Zellkultur als auch im Patientenorganismus, und um andererseits diese Zellen ggf. zu manipulieren, entweder durch den Antikörper selbst oder durch daran gekoppelte spezifische Reagenzien.

Die Erfindung betrifft ferner Hybridomzellen, die einen monoklonalen Antikörper gegen das Protein MGC-24 erzeugen. Sie umfaßt insbesondere die Hybridomzellen, die unter der Nummer DSM ACC 2221 bei der Deutschen Sammlung von Mikroorganismen und Zellkulturen GmbH, DSM, hinterlegt sind und den Antikörper mit der Bezeichnung 103B2 produzieren.

Darüber hinaus betrifft die Erfindung auch ein Verfahren zur Herstellung von Hybridomzellen, die einen Antikörper gegen das native, unmodifizierte Zelloberflächenglykoprotein MGC-24 synthetisieren und freisetzen. Dieses Verfahren umfaßt die im Stand der Technik grundsätzlich geläufigen Schritte, wie sie bspw. von Bühring et al. in Hybridoma 1991, Band 10, Nr. 1, S. 77-78 beschrieben wurden:
1. Immunisierung oder Sensibilisierung eines Tieres, vorzugsweise einer Maus vom Balb/c-Stamm, mit dem Antigen bzw. Immunogen;
2. Gewinnung der antikörperproduzierenden Zellen, vorzugsweise der Milzlymphozyten dieses Tieres;
3. Fusionierung dieser antikörperproduzierenden Zellen mit einer stabilen, immortalisierten Zellinie, vorzugsweise einer Myelomzellinie, zu Hybridomzellen; und
4. Vereinzelung und Vermehrung (Klonierung) solcher Hybridomzellen, die einen Antikörper sezernieren, der an das Antigen bindet.

Das erfindungsgemäß gefundene Verfahren zeichnet sich dadurch aus, daß das Tier mit Zellen der undifferenzierten, megakaryoblastären Zellinie MOLM-1 immunisiert wird.

Dabei erwies sich als Vorteil, daß diese Zellinie eine starke Expression von MGC-24 zeigt, wie sich während der zu dem Antikörper 103B2 führenden Versuche zeigte.

Wenn Hybridomzellen gesucht werden, die stammzellspezifische Antikörper produzieren, ist es bei der Vereinzelung der Hybridomzellen bevorzugt, wenn solche Hybridomzellen ausgewählt werden, die Antikörper mit einer Spezifität für Knochenmarkzellen produzieren, wobei es ferner bevorzugt ist, wenn nur solche Hybridomzellen auf die Spezifität der von ihnen produzierten Antikörper mit Knochenmarkzellen getestet werden, bei denen zuvor nachgewiesen wurde, daß diese Antikörper eine schwache oder bevorzugt eine negative Reaktion mit peripheren Blutzellen zeigen.

Hier ist von Vorteil, daß ein schnelles Screening möglich ist, ohne das viele Zellen vergebens getestet werden müssen. Es wurde nämlich überraschenderweise gefunden, daß MGC-24 auch auf Stammzellen exprimiert ist, so daß bei dem Screening die Tatsache ausgenutzt werden konnte, daß im Knochenmark verstärkt undifferenzierte Zellen einschließlich von hämatopoetischen Stamuzellen vorkommen, die das von dem Antikörper zu erkennende Antigen aufweisen. Durch den Vortest auf Reaktion mit peripheren Blutzellen können dabei solche Antikörper auf einfache Weise gewonnen werden, die selektiv an Antigene auf Knochenmarkzellen binden und nicht bzw. nur schwach auf peripheren Blutzellen exprimiert sind. Mit anderen Worten, die Spezifität bei der Auswahl wird hierdurch auf einfache Weise deutlich erhöht.

Die Erfindung betrifft auch die Verwendung eines monoklonalen Antikörpers gegen das Zelloberflächenglykoprotein MGC-24 zur diagnostischen und/oder therapeutischen Behandlung von Tumoren, insbesondere von Magen- und Coloncarcinomen.

Tumorzellen, insbesondere Zellen von Magen- und Coloncarcinomen, zeichnen sich durch einen vergleichsweise hohen Gehalt an Zelloberflächenglykoprotein MGC-24 aus. Ein erfindungsgemäßer Antikörper , der mit einem Nachweismittel, beispielsweise einem radioaktiven Marker, gekoppelt ist, bindet dieses Nachweismittel indirekt an diese Zellen und ermöglicht damit den direkten Nachweis dieser Zellen, beispielsweise mit röntgendiagnostischen/szintigraphischen Methoden. Damit ist eine sehr frühe Tumordiagnose unter Umständen sogar in-vivo möglich.

In entsprechender Art und Weise kann der Antikörper mit einem therapeutisch wirksamen Mittel gekoppelt sein und dadurch eine direkte und gezielte Beeinflussung oder gar Eliminierung von MGC-24 tragenden Zellen, insbesondere Tumorzellen, ermöglichen.

In einer bevorzugten Ausführungsform wird ein Antikörper, der von den unter der Nummer DSM ACC 2221 bei der Deutschen Sammlung von Mikroorganismen und Zellkulturen GmbH, DSM, hinterlegten Hybridomzellen produziert und freigesetzt wird, zu einer solchen diagnostischen und/oder therapeutischen Behandlung verwendet.

Um die therapeutische und/oder diagnostische Applikation des erfindungsgemäßen Antikörpers zu erleichtern, kann der Antikörper mit entsprechend geeigneten Hilfssubstanzen in einer pharmazeutischen Zubereitung vermischt sein. Die Erfindung betrifft deshalb auch ein pharmazeutisches Mittel zur diagnostischen und/oder therapeutischen Behandlung von Tumoren, das einen erfindungsgemäßen, an das Zelloberflächenglykoprotein MGC-24 bindenden Antikörper enthält. Vorzugsweise enthält dieses pharmazeutische Mittel einen Antikörper, wie er von den unter der Nummer DSM ACC 2221 bei der Deutschen Sammlung von Mikroorganismen und Zellkulturen GmbH, DSM, hinterlegten Hybridomzellen produziert und freigesetzt wird.

Mit einem erfindungsgemäßen Antikörper können MGC-24 tragende Zellen aus einer Suspension verschiedenartiger Zellen mit den im Stand der Technik bekannten Testverfahren, wie z.B. dem Enzyme linked immunosorbet assay, kurz ELISA, oder dem Radioimmunoassay, kurz RIA, nachgewiesen werden. Die vorliegende Erfindung betrifft deshalb auch einen Kit zum Nachweis von Peanut Agglutinin (PNA)-bindendem Zelloberflächenglykoprotein, der einen monoklonalen Antikörper umfaßt, der spezifisch an das native Glykoprotein MGC-24 bindet.

Eine bevorzugte Ausführungsform dieses Kits ist dadurch gekennzeichnet, daß der Kit einen Antikörper umfaßt, wie er von den unter der Nummer DSM ACC 2221 bei der Deutschen Sammlung von Mikroorganismen und Zellkulturen GmbH, DSM, hinterlegten Hybridomzellen erzeugt wird.

Im Rahmen der vorliegenden Erfindung wurde - wie bereits erwähnt - überraschenderweise gefunden, daß der monoklonale Antikörper mit der Bezeichnung 103B2, der von den unter der Nummer DSM ACC 2221 bei der Deutschen Sammlung von Mikroorganismen und Zellkulturen GmbH, DSM, hinterlegten Hybridomzellen erzeugt wird, an Stammzellen bindet.

Die Erfindung betrifft deshalb auch die Verwendung eines erfindungsgemäßen Antikörpers, vorzugsweise des Antikörpers 103B2, zum Nachweis von hämatopoetischen Zellen, sowie einen Kit zum Nachweis von hämatopoetischen Zellen, der einen erfindungsgemäßen Antikörper, vorzugsweise den Antikörper 103B2, enthält. Dadurch ist es möglich, undifferenzierte CD 34⁺ Subpopulationen aufzutrennen und für funktionelle Analysen Zellen der erythroiden Reihe bzw. aus dem Knochenmark zu selektieren und aufzureinigen.

Als weiterer überraschender Effekt wurde gefunden, daß die Zugabe von monoklonalem Antikörper 103B2 zu unausgereiften erythroiden Zellen die in-vitro Hämatopoese hemmt.

Die Erfindung betrifft deshalb auch die Verwendung eines erfindungsgemäßen Antikörpers, vorzugsweise des Antikörpers 103B2, zur Hemmung der Hämatopoese.

Weitere Vorteile ergeben sich aus der nachstehenden Beschreibung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird im folgenden anhand von Anwendungs- und Ausführungsbeispielen unter Heranziehung der Figuren näher erläutert.

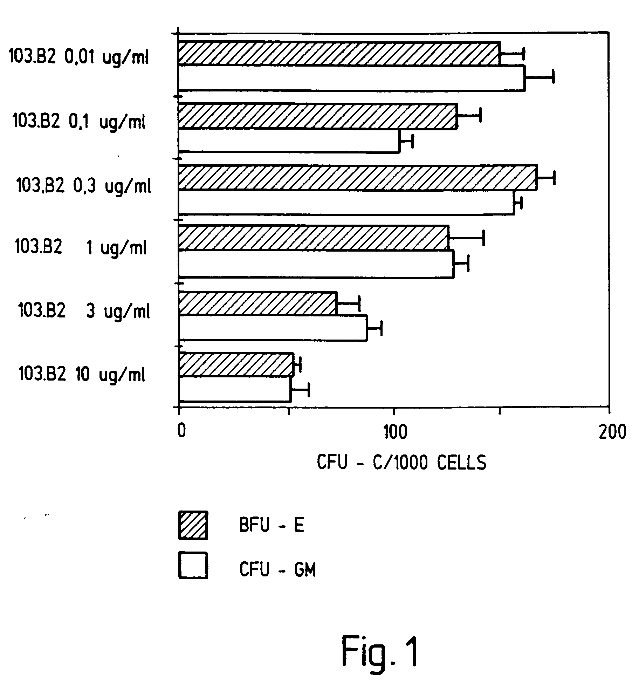

- Fig. 1: ist ein Balkendiagramm, das die Hemmung der Hämatopoese durch den Antikörper 103B2 zeigt.

### Beispiel 1: Herstellung und Charakterisierung von monoklonalen Antikörpern gegen das Zelloberflächenglykoprotein MGC-24

Als Antigen werden Zellen der undifferenzierten, megakaryoblastären Zellinie MOLM-1 verwendet (Matsuo Y, Adachi T, Tsubota T, Imanishi J, Minowada J. Establishment and characterization of a novel megakaryoblastoid cell line, MOLM-1, from a patient with chronic myelogenous leukemia. Human Cell 1991; 4: 261-264).

Acht Wochen alte Balb/c-Mäuse werden zweimal in Intervallen von 10 Tagen intraperitoneal mit 10⁷ Zellen der Zellinie MOLM-1 immunisiert. Vier Tage vor der Fusion werden 5 x 10⁵ Zellen direkt in die Milz appliziert, um die Immunantwort zu verstärken.

Die Antikörper-Bildung im Mausorganismus wird dadurch überprüft, daß das Blutserum des betreffenden Tieres in dem dem Fachmann geläufigen ELISA-Test auf Bindungseigenschaften mit dem Antigen untersucht wird.

Nach ca. 3 Wochen werden die Lymphozyten des erfolgreich immunisierten Tieres gewonnen, indem die Milz herausoperiert und zu einer Zellsuspension zerkleinert wird.

Die suspendierten Milzzellen werden in Anwesenheit von Polyethylenglykol mit Myelomzellen des bekannten Stammes SP2/0 fusioniert. Die Fusionskultur wird in Hypoxanthin-, Aminopterin- und Thymidin-(HAT-)haltigem Medium, hier in HAT-RPMI-1640, kultiviert, in dem sich nur Hybridzellen vermehren können, da diese sowohl die Eigenschaft der Myelomzellen zur unbegrenzten Teilungsfähigkeit als auch die Eigenschaft der Antikörper produzierenden Lymphozyten zum Wachstum in HAT-haltigem Medium haben.

Nach der Fusion werden die Zellen in Napfplatten ausplattiert und bei 37 °C, 5 % Co₂ inkubiert.

Die Kulturüberstände werden nach 10-14 Tagen auf der MOLM-1 Zellinie im Durchflußzytometer gescreent. In einem zweiten Schritt werden die Überstände auf Reaktivität mit peripheren Blutzellen getestet, da diese keine selektiven Stammzellantigene exprimieren. Überstände, die eine negative oder schwache Reaktion mit peripheren Blutzellen zeigen, werden anschließend auf Reaktivität mit Knochenmarkzellen getestet. Hybridome, die Antikörper mit Spezifität für Knochenmarkzellen produzieren, werden ausgewählt und nach dem bekannten Grenzverdünnungsverfahren vereinzelt und kultiviert, d.h. kloniert.

Bei dieser Screening-Strategie wird Nutzen aus der Tatsache gezogen, daß im Knochenmark verstärkt undifferenzierte Zellen einschließlich von hämatopoetischen Stammzellen vorkommen.

Positiv reagierende Hybridomzellkulturen werden weiter kultiviert, die Antikörper angereichert, gereinigt und charakterisiert.

Nach der obigen Screening-Strategie wurde der monoklonale Antikörper 103B2 erhalten. Die Isotype wurde über PE-konjugierte Anti-Isotyp-spezifische Antiseren durch direkte Immunfluoreszenz zu IgG3 bestimmt.

Die Produktion, Reinigung und Charakterisierung der Antikörper erfolgte mit den in Fachkeisen allgemein bekannten Methoden.

Der Antikörper 103B2, der von den unter der Nummer DSM ACC 2221 bei der Deutschen Sammlung von Mikroorganismen und Zellkulturen GmbH, DSM, hinterlegten Hybridomzellen erzeugt wird, weist die folgenden charakteristischen Merkmale auf:
- Immunglobulinklasse:: IgG3
- spezifische Bindungsaffinität an:: MGC-24

### Beispiel 2: Identifizierung des von dem monoklonalen Antikörper 103B2 erkannten Antigens.

Die Identifizierung des Antigens erfolgte über eine Stroma-Expressionsbibliothek.

Um das Gen zu isolieren, das für das Antigen codiert, das von dem monoklonalen Antikörper 103B2 erkannt wird, wurde eine retrovirale Expressionsbibliothek nach dem Verfahren erstellt, das von Rayner und Gonda in Mol.Cell.Biol. 1994, Band 14, Seite 880 beschrieben wurde. Bei dem hier verwendeten Verfahren wurde mRNA aus kultivierten Stromazellen des menschlichen Knochenmarks verwendet.

cDNA-Transkripte wurden direkt in den retroviralen Plasmid-Vektor pRUF.Neo kloniert. DNA aus der Bibliothek wurde verwendet, um eine amphotrope Wirtszellinie (PA317) zu transfizieren. Übergangsweise erzeugte retrovirale Partikel wurden geerntet und dazu verwendet, eine ecotrope Wirtszellinie stabil zu infizieren. Von diesen Zellen produzierte Viren wurden daraufhin verwendet, um die faktorabhängige hämatopoetische murine Zellinie FDC-P1 zu infizieren.

Infizierte FDC-P1-Zellen wurden bezüglich der G418-Resistenz ausgewählt, woraufhin Zellen isoliert und angereichert wurden, die das von dem Antikörper 103B2 erkannte Antigen exprimieren. Die Anreicherung von Zellen, die von dem Antikörper erkannt wurden, erfolgte dabei durch Einsatz mehrerer Durchläufe einer immuno-magnetischen Zellsortierung (Dynabeads). Nach dieser FACS-Sortierung wurden klonale Populationen der transfizierten Zellen etabliert.

Daraufhin wurden die proviralen cDNA-Inserts von genomischer DNA der infizierten Zellen zurückgewonnen. Zu diesem Zweck wurde eine PCR-Amplifikation durchgeführt, wobei spezifische retrovirale Primer verwendet wurden, die die Klonierungsstelle in dem Plasmidvektor flankieren. Auf diese Weise war es möglich, ein cDNA-Insert von ungefähr 3 kBp zu isolieren.

Eine Sequenzanalyse ergab, daß dieses Insert ein zuvor kloniertes Gen identifizierte, das zu der mucin-Familie gehört, nämlich das MGC-24, das von Masuzawa et al., J. Biochem. 112, 609-615 (1992), vollständig aufgeklärt wurde.

### Beispiel 3: Verwendung des monoklonalen Antikörpers 103B2 zur Hemmung der Hämatopoese in vitro

Um die Funktion der Antigene zu untersuchen, die von dem monoklonalen Antikörper 103B2 erkannt werden, wurde der Antikörper auf seine Fähigkeit hin untersucht, die hämatopoetische Zellentwicklung zu stören.

In einem ersten Versuch wurden gereinigte monoklonale Antikörper in zunehmenden Konzentrationen über den Bereich von 0,01-30 µg/ml zu halb-festen Klonierungslösungen von hämatopoetischen menschlichen Vorläuferzellen zugegeben. Diese Lösungen wurden unter Verwendung von normalen menschlichen Knochenmarkzellen hergestellt, die das CD34-Antigen exprimieren. Die Reinheit dieser Zellpopulation betrug in allen Versuchen 95-98,5 %.

Die CD34⁺-Zellen wurden für 14 Tage mit einer anfänglichen Konzentration von 1000 Zellen pro Milliliter in IMDM kultiviert, das durch L-Glutamin, Penicillin, Streptomycin, Beta-Mercaptoethanol, 0,9 % (Gewicht/Volumen) Methyl-Zellulose, 1 % Rinderserumalbumin, 30 % (Volumen/Volumen) fötales Kälberserum und 10 ng/ml der folgenden, gereinigten rekombinanten menschlichen Zytokine ergänzt wurde: IL-1, IL-3, IL-6, G-CSF, GM-CSF, SCF und Erythropoetin.

Nach 14 Tagen wurden die Kulturen in Übereinstimmung mit Standardkriterien nach der Anwesenheit von Kolonien durchgezählt, die sich aus Vorläuferzellen von Knochenmarkzellen (CFU-GM) oder erythroiden Zellen (BFU-E) ergaben. Verglichen mit einem bindenden (P4C2) und einem nicht bindenden (AA6) Kontrollantikörper vom gleichen Isotyp IgG3, der in gleichen Konzentrationen zugegeben wurde, führte der Antikörper 103B2 zu einer dosisabhängigen Inhibierung der Koloniebildung sowohl bei CFU-GM als auch bei BFU-E. Aus dem beigefügten Balkendiagramm in Fig. 1 ergibt sich, daß bei einer Konzentration von 3 µg/ml Antikörper 103B2 die Zahl der erythroiden Kolonien um ca. 50 % und bei einer Konzentration von 10 µg/ml um ca. 65 % der Ausgangszahl reduziert wird.

Als zweites wurde der Antikörper daraufhin untersucht, ob er die Hämatopoese in einer Stromazell-abhängigen Langzeitkultur stören kann. Der Antikörper 103B2 oder ein Kontrollantikörper wurden zu Kulturen von Stromazellen aus dem Knochenmark hinzugegeben. Diese Kulturen wurden aus Knochenmark hergestellt, das von normalen Spendern stammte. Die kultivierten Stromazellen wurden bestrahlt (1500 rad), um hämapoetische Zellen eine Woche vor dem Start des Experimentes abzutöten. Die Langzeitkultur wurde dann etabliert, indem 3 x 10⁴ CD34⁺-Zellen pro Kultur unter Anwesenheit der oben erwähnten Antikörper in einer Konzentration von 10 µg/ml hinzugegeben wurden.

Eine Woche nach der Zugabe der Antikörper konnte eine vollständige Blockierung der Hämatopoese in diesem System beobachtet werden, was durch das vollständige Fehlen von nachweisbaren hämatopoetischen Vorläuferzellen und durch den Tod der Stromazellen angezeigt wurde.

## Patentansprüche

1. Monoklonaler Antikörper, der spezifisch an das Peanut Agglutinin (PNA)-bindende Zelloberflächenglykoprotein MGC-24 bindet.

2. Monoklonaler Antikörper nach Anspruch 1, dadurch gekennzeichnet, daß er von Hybridomzellen produziert und freigesetzt wird, die unter der Nummer DSM ACC 2221 bei der Deutschen Sammlung von Mikroorganismen und Zellkulturen GmbH, DSM, nach dem Budapester Vertrag hinterlegt sind.

3. Hybridomzellen, dadurch gekennzeichnet, daß sie die Fähigkeit aufweisen, einen Antikörper gemäß einem der Ansprüche 1 oder 2 zu erzeugen.

4. Hybridomzellen nach Anspruch 3, dadurch gekennzeichnet, daß sie unter der Nummer DSM ACC 2221 bei der Deutschen Sammlung von Mikroorganismen und Zellkulturen GmbH, DSM, nach dem Budapester Vertrag hinterlegt sind.

5. Verfahren zur Herstellung von Hybridomzellen, die einen Antikörper nach einem der Ansprüche 1 oder 2 freisetzen, mit den Schritten:
1. Immunisierung oder Sensibilisierung eines Tieres, vorzugsweise einer Maus vom Balb/c-Stamm, mit dem Antigen bzw. Immunogen;
2. Gewinnung der antikörperproduzierenden Zellen, vorzugswseise der Milzlymphozyten dieses Tieres;
3. Fusionierung dieser antikörperproduzierenden Zellen mit einer stabilen, immortalisierten Zellinie, vorzugsweise einer Myelomzellinie, zu Hybridomzellen; und
4. Vereinzelung und Vermehrung (Klonierung) solcher Hybridomzellen, die einen Antikörper sezernieren, der an das Antigen bindet,
dadurch gekennzeichnet, daß das Tier mit Zellen der undifferenzierten, megakaryoblastären Zellinie MOLM-1 immunisiert wird.

6. Verfahren nach Anspruch 5, dadurch gekennzeichnet, daß bei der Vereinzelung solche Hybridomzellen ausgewählt werden, die Antikörper mit einer Spezifität für Knochenmarkzellen produzieren.

7. Verfahren nach Anspruch 6, dadurch gekennzeichnet, daß nur solche Hybridomzellen auf die Spezifität der von ihnen produzierten Antikörper mit Knochenmarkzellen getestet werden, bei denen zuvor nachgewiesen wurde, daß dieser Antikörper eine schwache oder bevorzugt eine negative Reaktion mit peripheren Blutzellen zeigt.

8. Verwendung eines Antikörpers nach einem der Ansprüche 1 oder 2 zur diagnostischen Behandlung von Tumoren, insbesondere von Magen- und Coloncarcinomen.

9. Verwendung eines Antikörpers nach einem der Ansprüche 1 oder 2 zur therapeutischen Behandlung von Tumoren, insbesondere von Magen- und Coloncarcinomen.

10. Pharmazeutisches Mittel zur diagnostischen Behandlung von Tumoren, dadurch gekennzeichnet, daß es einen Antikörper gemäß einem der Ansprüche 1 oder 2 enthält.

11. Pharmazeutisches Mittel nach Anspruch 10, dadurch gekennzeichnet, daß der Antikörper an ein zellulär zielgerichtetes Therapeutikum gekoppelt ist.

12. Pharmazeutisches Mittel zur therapeutischen Behandlung von Tumoren, dadurch gekennzeichnet, daß es einen Antikörper nach einem der Ansprüche 1 oder 2 enthält.

13. Pharmazeutisches Mittel nach Anspruch 12, dadurch gekennzeichnet, daß der Antikörper an ein zellulär zielgerichtetes Diagnostikum gekoppelt ist.

14. Kit zum Nachweis von Peanut Agglutinin (PNA)-bindendem Zelloberflächenglykoprotein, dadurch gekennzeichnet, daß er einen Antikörper nach einem der Ansprüche 1 oder 2 enthält.

15. Verwendung eines Antikörpers nach einem der Ansprüche 1 oder 2 zum Nachweis von hämatopoetischen Zellen.

16. Kit zum Nachweis von hämatopoetischen Zellen, dadurch gekennzeichnet, daß er einen Antikörper nach einem der Ansprüche 1 oder 2 enthält.

17. Verwendung eines Antikörpers nach einem der Ansprüche 1 oder 2 zur Hemmung der Hämatopoese.
